Europäisches Patentamt

European Patent Office

Office européen des brevets

(11) Publication number: **0 066 134**
**A1**

## (12) EUROPEAN PATENT APPLICATION

(21) Application number: **82104132.4**

(22) Date of filing: **12.05.82**

(51) Int. Cl.³: **A 61 G 7/00**
**A 61 G 13/00, A 61 G 15/00**
**A 47 C 1/027, A 47 C 19/00**
**A 61 B 6/10**

(30) Priority: **26.05.81 US 266702**

(43) Date of publication of application:
**08.12.82 Bulletin 82 49**

(84) Designated Contracting States:
**DE FR GB SE**

(71) Applicant: **SIEMENS AKTIENGESELLSCHAFT Berlin und München**
**Postfach 22 02 61**
**D-8000 München 22(DE)**

(72) Inventor: **Menor, George**
**6379 Creekview Court**
**Martinez California 94553(US)**

(72) Inventor: **Melson, Roger**
**2436 Buena Vista Avenue**
**Walnut Creek California 94596(US)**

(54) Wedge brake for a rotatable body.

(57) The brake is used for retaining a rotatable body such as a medical couch in a locked position. The brake contains two locking arms which are rotatable about a first and a second swivel axis, respectively. The end faces of these arms are formed as braking surfaces for engaging the aforementioned body in the locked position. The longitudinal axes of the locking arms form a wedge angle therebetween. A spring device is provided for retaining the locking arms in the locked position. There is also provided an electromagnetic device for acting against the forces of the spring device. When actuated, the electromagnetic device transfers the locking arms from the locked position to a released position wherein the braking surfaces are disengaged from the body. In this position, the body may be rotated.

FIG. 1

EP 0 066 134 A1

# BACKGROUND OF THE INVENTION

## 1. Field of the Invention

This invention relates to a brake for a rotatable body. In particular, this invention relates to a device for braking a rotation of a body, such as a treatment table or medical couch which is rotatable about a predetermined rotation axis.

## 2. Description of the Prior Art

In the medical field various apparatus are known which are rotated about a vertical and/or a horizontal axis in order to position a patient. In particular, a conventional medical couch or treatment table is provided with a base bearing which is rotated about a vertical axis in positioning of a patient. In such a medical couch a brake must be provided which is failsafe. That means that the brake must be released when a rotation is performed in order to position the patient and that the brake must be locked at all other times. Thus, the brake provides a safety feature in that the medical couch cannot be moved during treatment.

There are braking systems commercially available which are determined for locking rotatable bearings. These systems usually contain a sprocket and chain device for connecting the bearing to a first disk. This first disk acts together with a second disk when a braking operation is performed so that both disks provide for the necessary friction.

There are other brake systems commercially available which contain a band that surrounds a cylindrical device. Clamping the band to the cylindrical device will result in a friction sufficient for the braking process.

The prior art chain system incorporates a major problem. Usually there is some play in the chain which allows for an unwanted rotation of the base bearing and therefore of the treatment table when the brake system is supposedly locked. This would cause a movement of a patient who undergoes treatment. During irradiation treatment in radiotherapy, it is absolutely necessary that the rays of radiation impinge upon the morbid tissue and not the surrounding healthy tissue.

The prior art band system usually requires a high holding force along the band. This force can be provided by a lever system. Yet, this will result in a long stroke and therefore in a long actuation time. Other means are known, but it is difficult to achieve and maintain the necessary force.

-4-

## SUMMARY OF THE INVENTION

### 1. Objects

It is an object of this invention to provide a brake for a rotatable body which avoids play in the locked position.

It is another object of this invention to provide a brake for a rotatable body which avoids a chain or a band.

It is still another object of this invention to provide a relatively flat brake for a rotatable body.

It is still another object of this invention to provide a reliable brake for a medical couch.

### 2. Summary

According to this invention, a brake is provided which contains two locking arms. These locking arms are arranged to form a wedge-shaped or acute angle with each other. The end faces of the locking arms serve as braking surfaces.

In the locking position, two spring elements press the braking surfaces away from each other against the outer surface of the body to be locked.

Associated with each of the locking arms is a solenoid. Upon actuation of the solenoids, the locking arms are moved against the spring forces, that is the spring forces are overcome by the forces of the solenoids.

-5-

Thereby, the braking surfaces are released from the rotatable body.  The body, now free, may be rotated into another position.

Other objects, features and advantages of the invention will be apparent from the following more specific description of the preferred embodiments of the invention.

## BRIEF DESCRIPTION OF THE DRAWINGS

Fig. 1 is a plan view of a wedge brake according to this invention, and

Fig. 2 is a side or end view of the wedge brake of Fig. 1.

## DESCRIPTION OF THE PREFERRED EMBODIMENTS

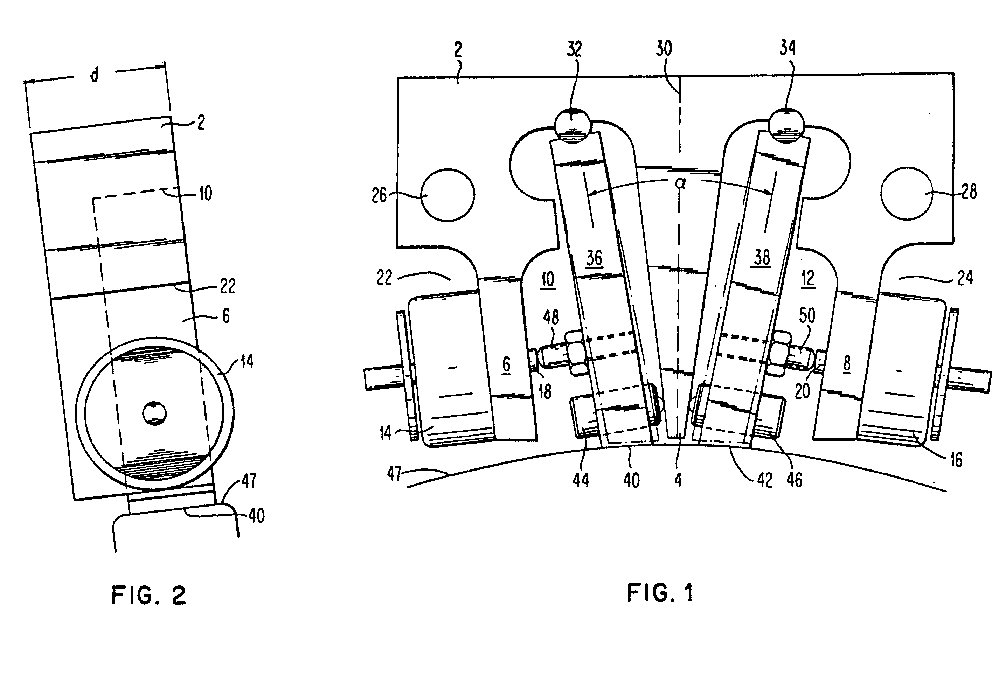

According to Figs. 1 and 2, a wedge brake is provided which contains a relatively flat housing 2. The thickness d is relatively small. The housing 2 contains a back wall, a center arm, center wall or center bar 4 and two side arms, side walls or side supports 6 and 8. The center bar 4 has the shape of a wedge. The side support 6 and the center bar 4 form a first chamber 10 in the space therebetween. Similarly, the space between the center bar 4 and the second side support 8 forms a second chamber 12.

On the outer surfaces of the side supports 6 and 8 are provided solenoids 14 and 16, respectively. The solenoid actuation pins 18 and 20 extend through the side supports 6 and 8, respectively, into the interior of the chambers 10 and 12, respectively.

The solenoids 14 and 16 are housed in recesses 22 and 24 in the end walls of the brake.

The housing 2 may be made of steel. Suitable holes 26 and 28 may be provided for attachment to a stationary base.

It will be noted that the brake is symmetrical with respect to a center line 30. All parts and components are provided twofold.

On the upper ends of the chambers 10 and 12 are provided round grooves for receiving cylindrical elements or pins 32 and 34, respectively. These pins 32 and 34 arranged parallel to each other represent pivoting axes.

Two locking arms or bars 36 and 38 are pivotly arranged in the chambers 10 and 12, respectively. Their longitudinal axes form a wedge or acute angle α. The locking arms 36 and 38 are each provided with a round recess or groove on their upper ends which recesses engage the cylinders 32 and 34, respectively. Thereby, the locking bars 36 and 38 are rotatable about the cylinders 32 and 34, respectively. The lower ends of the locking arms 36 and 38 form flat braking surfaces 40 and 42. These surfaces 40, 42 are inclined with respect to the longitudinal axes of the locking arms 36 and 38, respectively.

Spring plungers 44 and 46 are inserted by threaded engagement into the lower end portions of the locking arms 36 and 38, respectively. Their end or contact surfaces engage the side walls of the center bar 4. The spring forces are directed such that they press the locking arms 36 and 38 away from the center bar 4 and away from each other. Spring plungers 44 and 46 having a contact surface and being securable in threaded holes are commercially available. Due to the spring forces, the surfaces 40 and 42 engage firmly the surface 47 of a round rotatable body which is to be braked. The braking position is shown in Fig. 1 by solid lines.

A short distance above the spring plungers 44 and 46 are arranged threaded holes which contain setting screws 48 and 50, respectively. The outer ends of these setting screws 48 and 50 engage the pins 18 and 20 of the solenoids 14 and 16, respectively. Upon actuation of the solenoids 14 and 16, the pins 18 and 20 are pressed toward the center bar 4, thereby rotating the blocking arms 36 and 38 about the axes of the cylinders 32 and 34, respectively. Thereby, the spring force of each of the spring plungers 44 and 46 is overcome. This results in a disengagement of the braking surfaces 40 and 42 from the rotatable surface 47. This position of the blocking arms 36 and 38 is illustrated in Fig. 1 in dotted lines. When the activation of the solenoid 14 and 16 is terminated, the pins 18 and 20 will be retracted, and the locking arms 36 and 38 will return into their locking positions (see solid lines in Fig. 1) in which the braking surfaces 40 and 42 engage the rotating surface 47.

As mentioned above, the initial break force is provided by the spring plungers 44 and 46, respectively. When the braking surfaces 40 and 42 have engaged the rotatable surface 47, and when the rotatable body is pushed in any of its two rotation directions, an additional wedge action will begin. This wedge action increases the compression force between the braking surfaces 40 and 42 on the one hand, and the rotating surface 47 on the other hand. This supporting wedge action causes a secure locking.

It should be mentioned that it is also possible to release just one locking arm 36 or 38 at a time. This will allow rotation of the rotatable body in a preselected direction.

An essential advantage of the illustrated wedge brake is the fact that any attempt of a rotating action with

-10-

respect to the body will result in an enhancement of the braking force. Therefore, a secure and reliable braking device is obtained.

The wedge brake illustrated in Figs. 1 and 2 can be used with advantage in a medical couch used for radiation treatment of a patient. In such a case, the surface 47 is the surface of a bearing of the couch.

While the form of the wedge brake for a rotatable body herein described constitutes a preferred embodiment of the invention, it is to be understood that the invention is not limited to this precise form of assembly, and that a variety of changes may be made therein without departing from the scope of the invention.

WHAT IS CLAIMED IS:

1.  A brake for retaining a rotatable body in a locked position, comprising in combination

    a)  two locking arms rotatable about a first and a second swivel axis, respectively, whereby the end faces of said arms are formed as braking surfaces for engaging said body in said locked position, the longitudinal axes of said locking arms forming a wedge angle therebetween,

    b)  spring means for retaining said arms in said locked position, and

    c)  electromagnetic means for acting against said spring forces, thereby transferring said locking arms from said locked position to a released position wherein said braking surfaces are disengaged from said body.

2.  The brake according to claim 1, wherein said locking arms are metal bars, and wherein said swivel axes are located on one end respectively of said bars.

3.  The brake according to claim 1, wherein a housing is provided having a center wall and a first and a second side wall, wherein the space between said first side wall and said center wall form a first chamber, wherein the space between said second side wall and said center wall form a second chamber, wherein said first locking arm is positioned within said first

-12-

chamber, and said second locking arm is positioned
within said second chamber.

4. The brake according to claim 1, wherein said
center wall is a tapered wall.

FIG. 2

FIG. 1

| | European Patent Office | EUROPEAN SEARCH REPORT | Application number |
|---|---|---|---|
| | | | EP 82 10 4132 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-3 085 662 (BINDER) * column 1, lines 11-34, 63-67; column 2, lines 1-55; figure 3 * | 1 | A 61 G 7/00 A 61 G 13/00 A 61 G 15/00 A 47 C 1/027 A 47 C 19/00 A 61 B 6/10 |
| A | * column 1, lines 67-72 * | 2 | |
| A | US-A-2 341 465 (MONNOT) * page 2, column 1, lines 24-75; column 2, lines 1-9; figures 2,3 * | 1,2 | |
| A | US-A-3 382 960 (WILLEY) | | |
| A | US-A-3 866 722 (KJOS) | | TECHNICAL FIELDS SEARCHED (Int. Cl. ³) |
| A | US-A-3 230 714 (REDFIELD) | | A 61 G A 61 B A 47 C F 16 D |

The present search report has been drawn up for all claims

| Place of search THE HAGUE | Date of completion of the search 07-09-1982 | Examiner MAROSCIA A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82